# EUROPEAN PATENT SPECIFICATION

(11) **EP 1 044 372 B1**
(45) Date of publication and mention of the grant of the patent: **03.09.2003**
(21) Application number: 98965359.7
(22) Date of filing: 30.12.1998
(51) Int. Cl.: G01N 33/53

(54) **Method for determining an analyte in a sample using a flow matrix which comprises addition of reagents at two or more positions of the matrix**
Verfahren zur Bestimmung eines Analyts in einer Probe mittels einer Durchflussmatrix bei dem die Reagenzienzugabe an zwei oder mehreren zusätzlichen Stellen der Matrix stattfindet
Procedé de détermination d'un analyte dans un échantillon utilisant une matrice qui permet l'écoulement de liquide comprenant l'addition de réactives dans deux ou plusieurs positions de la matrice

(30) Priority: 30.12.1997 SE 9704934
(43) Date of publication of application: 18.10.2000
(73) Proprietor: PHARMACIA DIAGNOSTICS AB, 751 82 Uppsala (SE)
(72) Inventor: MENDEL-HARTVIG, Ib, S-756 55 Uppsala (SE); ZELIKMAN, Ilya, S-743 32 Storvreta (SE); RUNDSTRÖM, Gerd, S-752 41 Uppsala (SE)
(74) Representative: Tannerfeldt, Sigrid Agneta
(86) International application number: SE9802463
(87) International publication number: WO99036776

(56) References cited:
- EP-A2- 0 306 336
- WO-A1-91/14942
- WO-A1-92/02818

## Description

### Technical field

The invention relates to a method for determination of an analyte in a sample by use of biospecific affinity reactants (Reactant 1, Reactant 2 etc.), one of which is analytically detectable (Reactant*) and one is firmly anchored in a detection zone in a transport flow matrix (Reactant I). The sample (analyte) is transported by a flow in the matrix from one application zone for liquid (LZS) containing the analyte (sample) and/or a buffer, to the detection zone (DZ), in which Reactant I is firmly anchored. At the same time as the sample is transported in the matrix the soluble reactants, including Reactant*, are also being transported. In the detection zone Reactant* is captured in an amount which is related to the amount of analyte present in the sample. To achieve this, Reactant* is chosen so that it may bind biospecifically directly to Reactant I or indirectly via one or more added biospecific affinity reactants (including the analyte). The amount of analyte is then determined from the amount of Reactant* bound in the detection zone. The transport flow may contain zones, in which different biospecific affinity reactants (e.g. Reactant*, but not analyte) have been applied in advance (predeposited) in order to be dissolved and transported along with the flow towards the detection zone.

By reactants (including the analyte), exhibiting biospecific affinity (bioaffinity reactants), are meant individual members in the reactant pairs: antigen/hapten - antibody; biotin - avidin/streptavidin; two complementary single chains of nucleic acid etc. As antibodies antigen binding antibody fragments such as Fab, F(ab)₂', single chain Fv antibodies (scFv) etc. are considered. Relevant reactants do not have to be naturally occurring but can also be synthetically produced molecules/binders.

The type of test methodology in question has previously been used primarily for biospecific affinity reactants where at least one part of an employed reactant pair has exhibited protein structure, in particular in connection with so called immunochemical determination processes.

The biospecific affinity reactions are primarily performed in aqueous media (e.g. water).

The technique in question is well known and has often been applied to so called test strips, where the strip has functioned as a flow matrix. The flow has been initiated in the zone to which the sample has been added (LZS). The flow has often been lateral, i.e. parallel to the surface of the matrix, or of other types, e.g. in depth in the matrix.

The test protocols have been of so called inhibition type (competitive) or non-inhibition type (non-competitive, sandwich). See e.g. Behringwerke US 4,861,711; Unilever WO 88/08534; Abbot US 5,120,643; Becton Dickinson EP 284,232 and US 4,855,240; Abbot/Syntex US 4,740,468; Pharmacia AB, WO 96/22532, etc.

In this context one often mentions simultaneous and sequential methods (protocols) regarding certain reactants (especially analyte and Reactant*). In simultaneous variants analyte (sample) and the relevant reactant, e.g. Reactant*, are transported simultaneously into the detection zone. Simultaneous variants may be obtained, if a sample is pre-incubated/mixed with Reactant* or if Reactant* has been predeposited in the sample application zone or in a zone downstream of the sample application zone but before the detection zone. In sequential variants the analyte (sample) is transported before a reactant, e.g. Reactant*, into the detection zone. Sequential variants may be obtained, if the relevant reactant, e.g. Reactant*, is added to the same application zone as the sample after the sample (analyte) has been transported out of the zone. A variant of sequential methodology is discussed in US 4,855,240 (Becton & Dickinson). As an alternative to the sample (analyte) being transported before "tracer" (=Reactant*) in the same transport flow, US 4,855,240 describes separated transport flows, in which the time of transport is regulated such that sample (analyze) reaches the detection zone before the "tracer" (Reactant*). Another variant of sequential methodology is described in Syntex (U.S.A.) Inc. EP 306,336, where a device comprising a housing provided a first means for introducing a sample and a second means for introducing a liquid reagent other than the sample, is used.

The term simultaneous tests has often included every variant, in which sample and Reactant* are pre-incubated/mixed before being added to a flow matrix or in which sample is added to a flow matrix, in which Reactant* is predeposited in the sample application zone or downstream thereof. The term sequential tests has similarly included every variant, in which Reactant* is added to the sample application zone after the sample has migrated out of its application zone. Thus, considerations have not been taken, concerning if the order of analyte and Reactant* is changed during transport to the detection zone. If not otherwise stated, this nomenclature is also used for the present invention, but is now adapted so that there are several application zones for liquid. This view means that primarily the initial order is considered, when both analyte and Reactant* are in soluble form, and not the order in which analyte and Reactant* are transported into the detection zone.

### Disadvantages with prior art and objects of the invention.

The prior art has often involved practical problems on automation, primarily because pre-incubation or sequential addition of sample and reactants have often been required, often in a certain predetermined order defined by the test protocol used. The object of the invention is to (a) facilitate automation, (b) avoid sequential addition of sample and the analytically detectable reactant (Reactant*), and (c) allow for pre-deposited Reactant* when using sequential methodology, which relates to analyte and Reactant*. More general aims are to achieve high quality test results, preferably with improved sensitivity and precision than given by previous variants.

### The invention

Surprisingly we have now discovered that if flow is initiated by almost simultaneous addition of liquid to two adjacent zones in a flow matrix, the liquid added in the downstream zone migrates before the liquid which has been added in the upstream zone in direction towards the detection zone. Our discovery involves that zonewise migration of liquids may also be obtained if addition of liquid in an upstream zone is performed after addition of liquid in the nearest downstream zone. By applying this discovery on the relevant type of analysis methods, improvements can be obtained regarding the objects stated above.

A first main aspect of the invention relates to the initially mentioned analysis methods and is characterized in that
A. the flow matrix exhibits at least two application zones for liquid arranged substantially adjacent to each other: 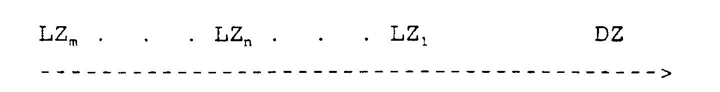 wherein
   a) LZₙ is an application zone for liquid, where n is the position of the application zone LZₙ (n is an integer 2 < n ≤ m)
   b) m is the total number of application zones, in which flow is initiated,
   c) one LZₙ is an application zone for sample (LZ_{n'}S) and one LZₙ is application zone for Reactant* (LZ_{n''}R*) with n'' ≥ n',
   d) -----> is the direction of the flow, and
   e) DZ is detection zone, and
B. flow is initiated by adding liquid to each zone
LZₘ . . LZₙ . . LZ₁ in such a way that liquidₙ₊₁, added to the application zone LZₙ₊₁, is transported through the matrix after liquidₙ, added to the nearest downstream application zone LZₙ.

Liquidₙ₊₁ may easily migrate immediately after liquidₙ, if the corresponding zones for application of liquid are adjacent to each other or if added liquid volumes are adapted for this aim.

In the most common case the above mentioned involves adding liquidₙ₊₁ to LZₙ₊₁ before or primarily simultaneously with adding liquidₙ to LZₙ. For n = m, LZₙ₊₁ is lacking, and for that zone it is therefore not possible to add any liquid to LZₘ₊₁. Practical advantages are achieved if the addition is performed primarily simultaneously for all LZₘ . . LZₙ . . LZ₁.

The number (m) of application zones for liquid (LZₘ . . LZₙ . . LZ₁) may in principle be any number with the exception of one (m ≠ 1). For practical reasons it is likely that in the future 2 ≤ m ≤ 10, preferably 2 ≤ m ≤ 6, such as m = 2 or 3 or 4 or 5.

The liquids added (liquid₁ ... liquidₘ) may consist of only buffer solution or buffer solution plus a reactant (Reactant 1, Reactant 2 etc.), needed to make it possible for Reactant* to be captured in the detection zone in an amount related to the amount of analyte in the sample. Also Reactant* may be included in a liquidₙ. As a rule the composition of transported components from an application zone is not the same as from the nearest adjacent application zone, in which flow is initiated (LZₙ₊₁ and LZₙ₋₁ with the exception of n = m and n = 1 for which the zones LZₙ₊₁ and LZₙ₋₁, respectively, are lacking) .

By the expression "substantially adjacent to each other" is meant that the application zones for liquid are immediately adjacent to each other or with an intermediate area of matrix which preferaby is no more than about 2 mm, and particularly no more than about 1 mm.

A liquid added in an application zone may have a tendency to spread on top of the matrix to parts of the matrix being outside the zone. For adjacent zones this means that liquids may be mixed with each other in an undesired way. To avoid this, physical barriers delimiting two adjacent application zones (zone spacers) are placed. The barriers should primarily be placed on top of the matrix, but may be extended down into the matrix without completely quenching the flow. Delimitation is primarily against an adjacent zone for application of liquid, but can of course extend around a whole application zone for liquid. Liquid may also be introduced via pads or material layers applied on the matrix and from the same or a different material than the matrix material. In such as case there is no need for zone spacers.

Relevant reactants may be predeposited in an application zone for liquid (LZₙ) or between two such zones. An application zone for liquid, only intended for transporting buffering components and/or other components not participating in the biospecific affinity reactions (i.e. liquid neither containing nor intended for transport of any reactant or analyte), is called LZₙB below. An application zone for liquid (LZₙ), where the liquid contains a reactant or is intended for transport of a reactant, e.g. Reactant*, Reactant 1, Reactant 2 etc., is called LZ_{n''}R*, LZ_{n'''}R1, LZ_{n''''}R2 etc. below. If a liquidₙ is to transport a combination of components, e.g. Reactant* and analyte (sample) the application zone will be common for the components and will be designated LZ_{n'''}R2/R1 etc. For the combination sample and Reactant*, the application zone will be LZ_{n'}R*/S (n' = n''). That liquidₙ is intended for transport of a certain reactant includes that the reactant in question also can be predeposited in the zone LZₙ. The latter includes that the reactant may be predeposited in an area downstream of the application zone for the relevant liquid but upstream of the nearest downstream located LZ (LZₙ₋₁), or if n = 1 only upstream of the detection zone (as LZₙ₋₁ then is lacking).

By predeposition is meant that a reactant is added in advance to the matrix and in a way so as not to spread in the matrix until it is reached by liquid, which has been applied to initiate flow. Predeposition of reactants may be performed in a way known per se. (See e.g. Behringwerke US 4,851,711; Unilever WO 88/08534; Abbot US 5,120,643; Becton Dickinson EP 284,232). It is important that arrangements are made so that the reactant in question is quickly dissolved, when liquid passes through an area, containing predeposited reactant. In order to achieve quick dissolution it has been common to incorporate reactants in substances that as such dissolve quickly. This type of substances are often hydrophilic with polar and/or charged groups, such as hydroxy, carboxy, amino, sulphonate etc. In particular there may be mentioned hydrophilic quickly soluble polymers, e.g. having carbohydrate structure, simple sugars including mono-, di- and oligosaccharides and corresponding sugar alcohols (mannitol, sorbitol etc.). It is common practice to first coat the application zone in question with a layer of the quickly soluble substance, and then the reactant is applied, optionally followed by one additional layer of quickly soluble substance. An alternative way is by incorporating the reactant in particles of quickly soluble material which then are deposited in the relevant zone of the matrix.

Some of the most important embodiments regarding the application zones for liquid may be summarized: 2 ≤ m ≤ 6; n' is 1, 2 or 3; n'' > n' or n'' = n'; LZ_{n'}S is the application zone for sample and optionally also for Reactant* or other reactant; LZₙ'₊₁, LZₙ'₊₂, LZₙ'₊₃, LZₙ'₋₁, and LZₙ'₋₂ are application zones for liquids intended for transport of Reactant* or other reactant or buffer without reactant as far as allowed by m, n'' and n'.

Transport flow through the particular types of matrix may be achieved by the action of capillary forces, e.g. by starting with a substantially dry matrix. A sucking body may be placed at the end of the flow as an aid. Flow, meaning transport of primarily only dissolved components, may be achieved if an electrical field is applied across the matrix.

### Test protocols

By use of the invention reactants and analyte can be made to migrate zonewise as individual components or together in different combinations towards the detection zone. The exact sequence of application zones is determined by the test protocol to be utilized.

The invention may be applied to competitive (inhibition) as well as non-competitive (non-inhibition) test variants irrespective of if these are simultaneous or sequential regarding any reactant. Illustrative systems are shown schematically below in form of the complexes formed. "-" relates to firm anchoring to the matrix, "---" relates to binding via biospecific affinity. For the sake of simplicity it has been assumed that reactants used are monovalent regarding utilized binding sites.

### A. Sandwich protocol (non-inhibition)

Reactant I and Reactant* both have biospecific affinity for the analyte. x = number of moles of Reactant I on the matrix and y = number of moles of analyte (= number of moles of Reactant*), bound to Reactant I.

Complex in the detection zone:
Matrix (-Reactant I)_{x-y} (-Reactant I --- analyte --- Reactant*)_{y}

### Simultaneous variants:

- m = 2:: LZ₂R*/S LZ₁B DZ

### Sequential variants :

- m = 2:: LZ₂R* LZ₁S DZ.
- m = 3:: LZ₃R* LZ₂B LZ₁S DZ and alternatives where the buffer zone has position 1 or 3.
- m = 4:: LZ₄B LZ₃R* LZ₂B LZ₁S DZ and alternatives where any of the buffer zones is placed in position 1.
- m = 5:: The same sequence as for m = 4 with the exception that an extra buffer zone is placed in position 1.

### B. Sandwich protocol (non-inhibition):

Reactant I exhibits biospecific affinity for Reactant II. Both Reactant II and Reactant* have biospecific affinity for the analyte. x = number of moles of Reactant I on the matrix, y = number of moles of analyte (= number of moles of Reactant*), bound to Reactant I via Reactant II. y + z is the number of moles of Reactant II bound to Reactant I.

Complex in the detection zone:
Matrix (-Reactant I)_{x-z-y} (-Reactant I --- Reactant II)_{z} (-Reactant I --- Reactant II--- analyte --- Reactant*)_{y}

### Simultaneous variants:

- m = 2:: The same as for protocol A with the exception that LZ₂R*/S is LZ₂R*/S/RII or that LZ₁B is LZ₁RII.
- m = 3:: LZ₃R*/S LZ₂B LZ₁RII DZ or LZ₃R*/S LZ₂RII LZ₁B DZ.

### Sequential variants:

- m = 2:: The same as for protocol A with the exception that LZ₁S is replaced by LZ₁S/RII.
- m = 3:: LZ₃R* LZ₂B LZ₁S/RII DZ or
LZ₃R* LZ₂S LZ₁RII DZ or
LZ₃R* LZ₂S/RII LZ₁B DZ.
- m = 4, 5, 6:: In analogy with protocol A sequences with up to 6 application zones for liquid may be considered.

### C. Inhibition protocol:

Reactant I is an analyte analogue, firmly anchored to the matrix, Reactant III exhibits biospecific affinity for the analyte and Reactant* has biospecific affinity for Reactant III. x = number of moles of Reactant I on the matrix. y = number of moles of Reactant III (= number of moles of Reactant*), bound to the matrix via Reactant I. The conditions are selected so that y is a measure of the amount of analyte in the sample.

Complex in the detection zone:
Matrix (-Reactant I)_{x-y} (-Reactant I --- Reactant III --- Reactant*)_{y}

### Simultaneous variants:

- m = 2:: LZ₂R*/RIII/S LZ₁B DZ.

### Sequential variants:

- m = 2:: LZ₂R* LZ₁/RIII/S DZ.
- m = 3, 4 and 5:: May be built up in analogy with protocol A.

### D. Inhibition protocol

Reactant I exhibits biospecific affinity for both Analyte and Reactant*. Reactant* is a soluble analyte analogue. x + y is the number of moles of Reactant I on the matrix, x and y are the number of moles of Reactant* and Analyte, respectively, being bound to the matrix.

Complex in the detection zone:
Matrix (-Reactant I --- Reactant*)ₓ (-Reactant I --- Analyte)_{y}:

### Simultaneous variant:

- m = 2:: LZ₂R*/S LZ₁B DZ

### Sequential variant:

- m = 2:: LZ₂R* LZ₁S DZ
- m = 3, 4 and 5: may be built up in analogy with protocol A.

### Matrices

The matrix defines the space in which the flow is transported. The matrix may be the inner surface of a simple flow channel (e.g. a capillary), the inner surface of a porous matrix having a system of flow channels (porous matrix) etc. extending through. For the sake of simplicity, matrices, usable in this variant of the invention, will be called flow matrices. Porous matrices may exist in the form of monoliths, sheets, columns, membranes, single flow channels having capillary dimensions or aggregated systems of such flow channels etc. They may also exist in the form of particles packed in column casings, compressed fibres etc. The inner surface of the matrices, i.e. the surface of the flow channels, should be hydrophilic, such that aqueous media (usually water) may be absorbed and transported through the matrices. The smallest inner dimension of the flow channels should be sufficiently large for allowing transport through the matrix of the reactants being used. The rule of thumb is that suitable matrices are selected among those having flow channels with the smallest inner dimension (often as a diameter for round channels) in the interval 0.4-1000 µm, preferably 0.4-100 µm if the matrix exhibits a system of mutually communicating flow channels. Flow channels having a smallest inner dimension in the upper part of the interval 0.4-1000 µm are primarily of interest for simple unbranched channels, through which flow is driven by an externally imposed pressure or suction.

Relevant matrices are often built up from a polymer, e.g. nitrocellulose, nylon etc. The material in the matrix as well as the physical and geometrical design of the flow channels may vary along the flow depending on what a certain part of the matrix is to be used for (Pharmacia AB WO 96/22532; Medix WO 94/15215).

### Detection zone

In the detection zone, Reactant I is firmly anchored to the matrix with bonds not allowing unintentional transport of Reactant I under the test conditions. Attachment of Reactant I to the matrix may be covalent, via physical adsorption, via biospecific affinity etc. Like prior art in this field the invention may utilize combinations of binding types, e.g. covalent binding to the matrix of a biospecific affinity reactant directed to Reactant I. In particular may be mentioned a matrix exhibiting a physically adsorptively or covalently bound member of a specific binding pair (reactant pair) in combination with Reaciant I coupled or conjugated to the other member of the specific binding pair, or a matrix exhibiting a similarly bound antibody directed to Reactant I. As examples of specific binding pairs may be mentioned immunological binding pairs, such as antigen-antibody and hapten-antibody, biotin-avidin or -streptavidin, lectin-sugar, hormone-hormone receptor, nucleic acid duplex. If reactant I binds to the matrix via another reactant according to the above, Reactant I need not be immobilized in the matrix but may either be movably (diffusively) predeposited in the matrix in an area or zone that is separated from the detection zone, or it may be added together with or separately from the sample. Anchoring of Reactant I to the matrix may be achieved via particles having been deposited in/on the matrix and to which Reactant I is covalently, physically adsorptively or biospecifically etc. bound. The particles attach to the matrix either because their size has been selected such that they cannot be transported through the matrix or via physical adsorption. See inter alia Abbott/Syntex US 4,740,468; Abbott EP 472,376; Hybritech EP 437,287 and EP 200,381; Grace & Co. EP 420,053; Fuji Photo Film US 4,657,739; Boehringer Mannheim WO 94/06012. For the invention the latter variant with smaller particles adsorbing physically to the matrix has been shown to be good.

In one and the same transport flow there may be several detection zones (DZ1, DZ2 etc.). One or more of the detection zones may relace to the same or different analytes. If the analytes are different Reactant I is usually different for each DZ.

### Analytically detectable reactant (Reactant*)

In the invention Reactant* cannot be an analyte. Usually analytical detectability is obtained because a natural reactant, e.g. an antibody or an antigen or a hapten, is provided with an analytically detectable group. Well known examples of often used groups are enzymatically active groups (enzyme, co-factor, co-enzyme, enzyme substrate etc.), fluorogenic, chromophoric, chemiluminiscent, radioactive groups etc. Groups being detected by means of a biospecific affinity reactant usually also are referred to this category, e.g. biotin, hapten, class-, subclass- and species-specific determinants in immunoglobulins etc.

A preferred label group is particles optionally containing any of the detectable groups above, such as fluorophoric groups or chromogenic groups (coloured particles). Useful particles often have a size in the interval of 0.001-5 µm. The particles may have colloidal dimensions, so called sol (i.e. usually spherical and monodisperse having a size in the interval 0.001-1 µm). Especially metal particles (e.g. gold sol), non-metal particles (e.g. SiO₂, carbon, latex and killed erythrocytes and bacteria) may be mentioned. Also particles of non-colloidal dimensions but with focus on non-sedimenting capability have been used. These have been more or less irregular and more or less polydisperse (carbon particles < 1 µm; Pharmacia AB, WO 96/22532).

For particles as label group reference is made to Unilever WO 88/08534; Abbott US 5,120,643; Becton Dickinson EP 284,232 among others.

In connection with the development that has led to the present invention it was surprisingly found that good results may be obtained if one simultaneously utilizes:
(a) Reactant* with label particles according to the above as a detectable group, and
(b) a detection zone, in which Reactant I has been anchored to the matrix via particles substantially having dimensions that would allow transport of the particles as such through the matrix.

We have achieved functioning systems, in which label particles and anchoring particles substantially have the same dimensions. This means with great probability that the label particles may be larger than the anchoring particles and vice versa, as long as they remain smaller than the flow channels defined by the matrix. The system may function with or without pre-deposition of Reactant* in the intended application zone. This embodiment is part of an invention, described in Pharmacia Diagnostics AB, WO 99/36780.

### Analytes

The invention is primarily adapted for determination of biospecific affinity reactants of the types initially mentioned. The reactants may be a cell or a virus or a part thereof. In particular antigen, such as an immunoglobulin or an antibody may be mentioned. For immunoglobulins the determination may relate to a certain Ig and/or certain Ig subclass. For antibodies the determination may relate to a certain specificity, optionally also the Ig class or the Ig subclass of the antibody. Relevant Ig classes are IgA, IgD, IgE, IgG and IgM. Relevant Ig subclasses are IgG1, IgG2, IgG3 and IgG4.

In sandwich variants (according to protocols A and B, above) the analyte may be an antibody, directed to an allergen/antigen/hapten and be derived from a certain species, a certain Ig class or a certain Ig subclass. In this case Reactant* may be an analytically detectable antibody directed to an epitope specific for the species, Ig class or Ig subclass and with Reactant I (protocol A) or Reactant II (protocol B) as the allergen/antigen/hapten. Alternatively the reverse may be selected, i.e. Reactant* is the allergen/antigen/hapten and Reactant I and Reactant II, respectively, is the antibody, directed to the analyte. When the analyte is an antigen in general, both Reactant I and Reactant* may be antibodies, directed to the antigen, in protocol A. For protocol B it is Reactant II and Reactant* that are antibodies directed to the antigen.

Competitive variants are most interesting for low molecular analytes. Illustrative examples are antigen and hapten. For protocol C Reactant I may be the antigen or the hapten, firmly anchored to the matrix, Reactant III may be an antibody, directed to the antigen, and Reactant* may be an antibody, directed to Reactant III. For protocol D Reactant I may be an antibody directed to the analyte and Reactant* may be the analyte labelled with an analytically detectable group.

The method of the invention may be performed as part of diagnosing allergy or autoimmune disease.

For the inventors it has been of special interest to measure anti-allergen antibodies of IgE or IgG class, for the latter preferably with focus on any of the subclasses mentioned. Measuring of allergen-specific antibodies can be utilized when diagnosing IgE mediated allergy.

### Samples

Relevant samples may be of biological origin, e.g. from different body fluids (whole blood, serum, plasma, urine, saliva, tear fluid, cerebrospinal fluid etc.), from cell culture media, processing procedures in biotechnology, from tissue extracts, from food stuff, from the environment (environmental analysis samples) etc. The samples may be pretreated in order to fit e.g. the matrix, the test protocol involved etc.

### Calibrators

Determination methods of the type that the invention is related to, involve that the detectable signal from the analytically detectable reactant (Reactant*) is measured and the measured signal (sample value) is taken as a measure of the amount of analyte present in the sample. To transfer the measurement signal to actual amounts of analyte the signal is usually compared to the corresponding signal (calibrator value) of known standard amounts of analyte (calibrators). In connection with the present invention a new calibrator system has been developed, which applied to the present invention constitutes a best embodiment.

This separate invention means that the used calibrator in advance has been anchored to a matrix (matrix calibrator), preferably of the same type as the one utilized for sample run. When measuring calibrator values, matrix calibrator is allowed to bind to Reactant*, and then the measurement signal from Reactant* is measured in a way known per se. By utilizing different amounts of matrix calibrator a series of calibrator values may be obtained corresponding to different pre-determined amounts of analyte in sample (standard amounts, dose-response curve, calibration curve).

Alternatively, instead a binder for the calibrator has been anchored to the matrix, and calibrator is added at the determination of calibrator value, optionally pre-deposited in the matrix upstream of the calibrator zone(s) to be dissolved by sample solution or buffer at the determination. When a calibrator binder is bound to the matrix, the calibrator may either be movably (diffusively) pre-deposited in the matrix in a zone separated from the detection zone, or be added together with or separately from the sample. The calibrator binder is usually one member of a specific binding pair (reactant pair), the other member of the binding pair being coupled or conjugated to the calibrator substance. As examples of such specific binding pairs may be mentioned immunological binding pairs such as antigen-antibody and hapten-antibody, biotin-avidin or -streptavidin, lectin-sugar, hormone-hormone receptor, nucleic acid duplex.

Applied to the present invention our new calibrator system involves primarily that the transport flow passes one or more zones with a calibrator, firmly anchored to the matrix in the respective calibrator zone (CZ).

Anchoring of a calibrator to the matrix in a calibrator zone may be performed according to the same principles as for anchoring of Reactant I to a detection zone.

Calibrator zones should be located downstream of an application zone for liquid, intended for transport of Reactant*. In relation to the detection zone (DZ) the calibrator zone is preferably located upstream.

Our invention relating to calibrators is described in detail in Pharmacia Diagnostics AB, WO 99/36777.

### A second main aspect of the invention

The flow matrix according to the above containing two or more application zones for liquid, optionally in the form of a kit wherein the flow matrix is comprised together with the analytically indicatable reactant, constitutes a second main aspect of the invention.

The invention is illustrated in the following non-limiting experimental part.

### EXAMPLE 1: SEQUENTIAL METHOD WITH THE ZONE SEQUENCE: LZ₄B, LZ₃R*, LZ₂B, LZ₁S, DZ. DETECTION OF hIgE IN TEST VARIANT WITH CARBON PARTICLE CONJUGATE

### Methods and materials

Adsorption of phenyldextran to polystyrene particles : Phenyldextran (substitution degree: 1 phenyl group on each fifth monosaccharide unit = 20%, Mw dextran 40,000, pharmacia Biotech AB, Uppsala, Sweden) was adsorbed to polystyrene particles (0.49 µm Bangs Laboratories, USA) by incubations under stirring with phenyldextran dissolved in deionized water to 1) 5 mg/ml, 10% particle suspension, RT 30-60 minutes; 2) 5 mg/ml, 5% particle suspension, RT 1 h; 3) 20 mg/ml, 1-2% particle suspension, RT 3 h or overnight. The particles were subsequently washed twice with deionized water. The particle suspensions were centrifuged between each incubation and wash (12,100xg, 25 min, Beckman, J-21, JA-20, 10,000 rpm). The particle suspension was finally sonicated (Ultrasonic bath, Branson 5210, 5 min).

Coupling of anti-human IgE antibody (anti-hIgE) to polystyrene particle: Anti-hIgE was coupled to phenyldextran coated polystyrene particles with CDAP (1-cyano-4-dimethylamino-pyridinium bromide (Kohn J and Wilchek M, FEBS Letters 154(1) (1983) 209-210).

Desalting and change of buffer of anti-hIgE was performed by gel filtration (PD-10, Pharmacia Biotech AB) in NaHCO₃, 0.1 M, pH 8.5. 2.3 g of polystyrene particles (coated with phenyldextran according to above) in 2% solution in 30% (by volume) acetone were activated with 17 ml CDAP (0.44 M) and 14 ml TEA (0.2 M triethylamine, Riedel-deHaën, Germany). CDAP was added with stirring for 150 seconds and TEA for 150 seconds. The particles were washed with 30% (by volume) acetone and centrifuged at 12,100xg (25 min, Beckman, J-21, JA-20, 10,000 rpm). 17 of anti-hIgE was coupled to the activated particles (2%, 0.15 g in 0.1 M NaHCO₃ pH 8.5) in incubating with stirring overnight at +4°C. The particles were centrifuged and decanted before deactivating with glutamic acid 0.05 M and aspartic acid 0.05 M in 0.1 M NaCHO₃ pH 8.5 when incubating with stirring overnight at +4°C. Coupled particles were washed once with 0.1 M NaHCO₃, 0.3 M NaCl, pH 8.5, once wich 0.1 M HAc, 0.3 M NaCl pH 5, once with 0.1 M NaHCO₃, pH 8.5 and once wich 20 mM borate buffer pH 8.5.

The concentration of particles was determined spectrophotometrically at A₆₀₀ nm with untreated particles as reference. Concentration of coupled protein was determined by having anti-hIgE present during the coupling and cpm measurement.

Carbon particle conjugate (Reactant*): This was prepared by anti-hIgE being adsorbed to carbon particles (sp100, < 1 µm, Degussa, Germany) according to WO 96/22532 (Pharmacia AB). The final solution used in the flow matrix contained 400 µg of carbon particles per ml.

Deposition of anti-hIgE-coupled particles on membrane: On nitrocellulose sheets (Whatman, 8 µm, length 5 cm and width 25 cm with polyester backing, anti-hIgE particles (prepared according to the above) were deposited in a zone over the width of the sheet (future detection zone) with Linear Striper (IVEK Corporation). The flow was 1 µl/sec and 1 µl/cm. The particles were diluted in borate buffer (20 mM, pH 8.5, Dextran T5000 4.2% w/w, sorbitol 5.8% w/w). The amount of deposited anti-IgE antibody was about 1000 ng/cm. The sheets were dried for 1 h at 30°C.

Zones for application of buffer, sample and carbon particle conjugate: Well separated from the zone, containing deposited material, 4 Inplastor strips (Mylar with glue on one side, Gelman) with a width of 1 mm were placed in parallel with the zone and parallel with each other at a distance of 5 mm from each other (zone spacers). The Inplastor strips thus defined four zones with a width of 5 mm. The sheets were cut perpendicularly relative to the zone containing deposited material, to strips with a width of 0.5 cm (the length of the strip then became 5 cm) (Singulator: Matrix 1201 membrane cutter, Kinematic automation). The final strips exhibited four parallel zones (application zones) separated by Inplastor strips as zone spacers and a separate zone with deposited anti-hIgE antibody (detection zone). As a comparison strips without zone spacers, i.e. without separated application zones were produced.

Test methodology: Strips with separated application zones were mounted on a plane holder. At the top (0.5 cm) of the strip (and with the detection zone as the nearest zone) a sucking membrane was placed (Whatman, 17 Chr, width 3 cm). The holder also gave a constant pressure on the sucking membranes. For simultaneous application of reagents to the four subzones a 4 channel Multipipette (Labsystems) was used. The multipipette was loaded so that serum sample (30 µl) was applied in the application zone nearest to the detection zone in the order of buffer (30 µl), carbon particle conjugate (30 µl) and buffer (30+30 µl) in the respective upstream application zone. For sequential application to the strips without zone spacers, 30 µl of sample were first applied to the lower end of the strip. After suction of sample volume, buffer (30 µl), carbon particle conjugate (30 µl) and buffer (30+30 µl) were added successively after suction. The carbon particle conjugate was prepared according to above and suspended in buffer. The buffer was NaPO₄ 0.1 M, BSA 3%, NaN₃ 0.05%, sucrose 3%, sodium chloride 0.2%, phenyldextran 0.05%, bovine gammaglobulin 0.05%, pH 7.5. The binding of the carbon particle conjugate to the detection zone was quantitated by measuring of absorbance (Ultroscan XL, Enhanced Laser Densitometer, LKB). IgE with standard concentrations in plasma environment (0; 0.5; 2; 10; 50, and 200 KU/L) was used as samples.

### Results

Having four application zones for liquid and simultaneous addition, the liquids were migrating in the order of the application zones, i.e. the sample being in the zone nearest to the detection zone was migrating first, without being mixed with the washing solution of the following application zone, which solution in turn started migrating, when the sample had been transported out of the application zone for samples. Correspondingly the liquids of the remaining zones were migrating sequentially without being mixed.

**Table 1:**

| Analysis results from runs with sequential addition in one zone and from simultaneous addition in four subzones (buffer, analytically detectable reactant, buffer, sample). | | |
|---|---|---|
| | Sequential addition in one zone | Simultaneous addition in 4 subzones |
| IgE KU/L | (Abs x1000) | (Abs x1000) |
| 0,5 | 0 | 12 |
| 2 | 312 | 207 |
| 10 | 1241 | 831 |
| 50 | 1921 | 1560 |
| 200 | 2115 | 2044 |

In **Table 1** is shown that the uptake decreases slightly for strips with discrete application zones compared to when addition is performed in one and the same zone. The decrease is, however, marginal. Therefore the experiment shows that generally the same result may be achieved if simulcaneous addition is performed to the zone sequence LZ₄B, LZ₃R*, LZ₂B, LZ₁S as if sample, Reactant* and buffer are added sequentially to a common application zone.

If a firmly anchored anti-IgE antibody (Reactant I) is replaced by an allergen a determination method for IgE antibodies directed to the allergen is obtained. Analogously, test systems related to antibodies of another class/subclass and with another specificity may be determined. Application zones for only buffer may be omitted. For additional alternative test protocols and analytes see above under the headings "Test protocols" and "Analytes".

### EXAMPLE 2: SEQUENTIAL METHOD WITH THE ZONE SEQUENCE: LZ₄B, LZ₃R*, LZ₂B, LZ₁S, DZ. DETECTION OF hIgE IN TEST VARIANT WITH FLUORESCENT DETECTION CONJUGATE

### Methods and materials

Coupling of anti-human IgE antibody (anti-hIgE) to a polystyrene particle: Anti-hIgE was coupled to polystyrene particles coated with phenyldextran (prepared according to Example 1) with CDAP (1-cyano-4-dimethylaminopyridinium bromide) (Kohn J and Wilchek M, FEBS Letters 154(1) (1983) 209-210). Desalting and buffer change of anti-hIgE was performed by gel filtration (PD-10, Amersham Pharmacia Biocech AB) in NaHCO₃, 0.1 M, pH 8,5.

0.35 g of polystyrene particles (2% solution) were activated with 5.2 ml of CDAP (0.44 M) and 4.2 ml of TEA (0.2 M triethylamine, Riedel-deHaën, Germany). CDAP was added with stirring for 60 seconds and TEA for 120 seconds. A five times excess of ice-cooled deionised water was added. The particles were centrifuged at 12,100xg (25 min., Beckman, J-21, JA-20, 10,1000 rpm). The resulting pellet was dissolved in ice-cooled, deionized water and washed once with ice-cooled deionized water and then centrifuged at 12,000 x g. 50 mg of anti-hIgE were coupled to the activated particles (2%, 0.35 g in 0.1 M NaHCO₃, pH 8.5). Incubation with stirring was the performed for 1 hour at +4°C. After centrifugation, the particles were deactivated with glutamic acid, 0.05 M, and aspartic acid, 0.05 M in 0.1 M NaHCO₃, pH 8.5. Incubation and stirring was then performed overnight at +4°C. Coupled particles were washed twice with 20 mM borate buffer, pH 8.5, whereupon the particle concentration was determined spectrophotometrically at A₆₀₀ₙₘ with untreated particles as reference. Coupled protein concentration was determined by having radioactive anti-hIgE present during the coupling.

Coupling of anti-hIgE antibodies to detection particles: Antibodies to hIgE cleaved with pepsin to fab'2 fragments were coupled to fluorescent polystyrene particles having aldehyde groups on their surface (Molecular Probes C-17177 TransFluoSphere, aldehyde-sulphate microspheres, 0.1 µm, 633/720, 2% solids). 23 mg of antibody were then coupled to 66 mg of particles in 50 mM NaPO₄, pH 6.5, overnight at room temperature, whereupon 205 µL of NaCNBH₄ (5 M) were added to reduce the coupling for 3 hours at room temperature. Centrifugation was performed at 20,800 x g for 50 minutes (50 minutes in Eppendorf 5417R, 14,000 rpm), and deactivation in glutamic acid, 0.05 M, and aspartic acid, 0.05 M, in deionized water, pH 6.5, was then performed overnight with stirring at room temperature. After centrifugation again at 20,800 x g, blocking was performed with 0.2% BSA in 50 mM NaPO₄, pH 7.4, with 0.05% NaN₃ and incubation was carried out overnight at +4°C. Centrifugation was then performed again at 20,800 x g and washing was performed twice with blocking buffer which was then used also for storage. The particle concentration was determined in a fluorimeter (Perkin-Elmer LS50B) with a standard curve prepared with the original particle. Coupled protein concentration was determined by having radioactive hIgE present during the coupling.

Deposition of anti-hIgE-coupled particles on membrane and application zones: Was performed according to Example 1, except that the Inplastor strips were replaced by strips of adhesive tape (2 mils clear polyester Arcare with glue on one side).

Test methodology: Strips with spaced application zones were mounted to an inclined plane, about 16°, in a holder. At the top (0.5 cm) of the strip (and with the detection zone as the nearest zone), two sucking membranes (Whatman, 17 Chr, width 3.4 cm) were placed on top of each other. The holder also exerted a constant pressure on the sucking membranes. For simultaneous application of reagents to the four subzones, a multichannel Finn pipette (Labsystems) was used. The multipipette was charged so that serum sample (30 µL) was applied to the application zone nearest to the detection zone in the order of buffer (15 µL), fluorescent particle conjugate (30 µL) and buffer (30 + 30 µL) in the respective application zone located upstream. For sequential application to the strips without zone spacer, 30 µL of sample were first applied to the lower end of the strip. After sucking of the sample volume, buffer (15 µL), detection particle conjugate (30 µL) and buffer (30 + 30 µL) were successively added after sucking. The particle conjugate was suspended in assay buffer consisting of NaPO₄ 0.1 M, BSA 3 %, NaN₃ 0.05 %, sucrose 10 %, NaCl, 0.15 M, bovine gammaglobulin 0.05 %, pH 7.5. The measurement of time started with the application of the sample, and the time until the last buffer had been sucked into the membrane was noted. The binding of the fluorescent particle conjugate to the detection zone was quantified by scanning with a red diode laser (635 ± 5 nm). As sample were used IgE standard concentrations in plasma environment (0, 0.5, 2, 10, 50 and 200 KU/L).

### Results:

Precisely as in Example i, the liquids migrated out of the application zone in the existing order. The time for a whole test with simultaneous application was about 20 minutes, and the time for a test with sequential application was about 25 minutes.

**Table 2:**

| Analysis results from runs with sequential application in one zone and from simultaneous application in four subzones (buffer, analytically detectable reactant, buffer, sample). | | |
|---|---|---|
| KU/L | Simultaneous addition to four subzones | Sequential addition to one zone |
| 0 | 0.048* | 0.038* |
| 0.5 | 0.053 | 0.047 |
| 2 | 0.085 | 0.074 |
| 10 | 0.286 | 0.256 |
| 50 | 1.334 | 1.291 |
| 200 | 2.507 | 2.487 |

| | | |
|---|---|---|
| * = scanning signal (Vmm) | | |

Table 2 shows that the uptake is comparable for strips with discrete application zones in comparison with addition to a single zone. The experiment therefore shows that the same result may be obtained if addition is made simultaneously to the zone sequence LZ₄B, LZ₃R*, LZ₂B, LZ₁S as if sample, Reactant* and buffer are added sequentially to a common application zone.

### EXAMPLE 3: SEQUENTIAL METHOD WITH THE ZONE SEQUENCE: LZ₅B, LZ₄R*, LZ₃B, LZ₂S, LZ₁B, DZ. DETECTION OF BIRCH-SPECIFIC hIgE IN TEST VARIANT WITH FLUORESCENT DETECTION CONJUGATE

### Methods and materials

Extraction of birch pollen allergen t3, Betula verucosa: 1 part (weight) of birch pollen (Allergon, Sweden) was extracted with 10 parts (volume) of 0.1 M phosphate buffer, pH 7.4. The extraction was continued for 2 hours on a shaker table at +4 °C. The extract was centrifuged at 4000 rpm for 1.75 hours. After filtration, the solution was applied to a PD-10 column (Pharmacia Biotech AB) and eluted in 0.1 M NaHCO₃, pH 8.5. The t3 eluate (designated: t3 extract 1/14) was taken to amino acid analysis for determination of the total protein content.

Coupling of birch pollen allergen to polystyrene particle : t3 extract was coupled to phenyldextran coated polystyrene particles (prepared according to Example 1) with CDAP. The coupling was effected analogously with the coupling of hIgE.

Polystyrene particles (2128 mg) coated with phenyldextran in 30 % (by volume) acetone, 2 % particle suspension, were activated with 954 mg of CDAP (100 mg/ml in 30 % acetone) and 7.63 ml of 0.2 M triethylamine (TEA, Riedel-de Haen, Germany). CDAP was added with stirring and TEA was added dropwise for 90 seconds and with stirring for totally 120 seconds. The reaction was stopped by the addition of 30 % acetone (4 times the volume) and centrifugation at 12,400 g for 35 minutes. The particles were washed once with deionized water.

640 ml of t3 extract 1/14 in 0.1 M NaHCO₃, pH 8.5, were added to the activated particles, and coupling was continued for 1 hour on a shaker table. After centrifugation, the particles were deactivated with 0.05 M aspartic acid and 0.05 M glutamic acid in 0.1 M NaHCO₃, pH 8.5. Incubation then took place on a shaker table overnight at +4 °C. The particles were then washed twice with 50 mM NaPO₄, 0.05 % NaN₃, pH 7.4. The particle concentration was determined spectrophotometrically at 600 nm with uncoated polystyrene particles as reference. t3-coupled polystyrene particles were taken to amino acid analysis for determination of the total protein content.

Deposition of t3-coupled polystyrene particles on a membrane: To nitrocellulose sheets with polyester backing (Whatman, 8 µm, 5 cm width) were applied zones of t3-coupled particles diluted to 4 % particle content in 50 mM NaPO₄, 10 % sucrose, 0.05 NaN₃, pH 7.4. The deposits were dried for 1 hour at 30 °C.

Zones for application of buffer, sample and detection particle conjugate : Five 1 mm wide strips of adhesive tape (2 mils clear polyester, Arcare with glue on one side) were placed well separated from the zone containing the deposited material and in parallel with the zone at a distance of 5 mm from each other. The tape strips thereby defined five different 5 mm wide zones. The sheets were cut perpendicularly to the zone containing deposited material to strips having a width of 0.5 cm (the length of the strip then became 5 cm) (Singulator: Matrix 1201 membrane cutter, Kinematic automation). The final strips exhibited five zones (application zones) separated by tape strips as zone spacers and a separate zone with deposited birch pollen (detection zone). Strips without zone spacers, i.e. without separated application zones, were prepared as a comparison.

Test methodology: Strips with separated application zones were mounted, and reagents were applied as in Example 2. Buffer (20 µL) was applied to the zone closest to the application zone, and then serum sample (30 µL), buffer (20 µL), detection particle conjugate (20 µL) and buffer (30 + 30 µL) in the respective application zone located upstream. For sequential application to the strips without zone spacer 20 µL of buffer were first applied to the lower end of the strip, and after sucking in thereof, 30 µL of sample were applied in the same position and then buffer (20 µL), fluorescent particle conjugate (20 µL) and buffer (30 + 30 µL). Before all applications, the preceding application had been sucked in by the strip. The detection particle conjugate and the buffer were according to Example 2.

### Results:

With the application zone consisting of five subzones and with simultaneous addition thereto, it turned out that the liquids, precisely as in the Examples above, migrated out of the application zone in the existing order. The time required for a whole test with simultaneous addition was about 21 minutes, and the time needed for a whole test with sequential addition was about 27 minutes.

**Table 3:**

| Analysis results from runs with sequential addition in one zone and from simultaneous addition in 4 subzones (buffer, analytically detectable reactant, buffer, sample). | | |
|---|---|---|
| | Simultaneous addition to 5 subzones | Sequential addition to 1 zone |
| neg | 0.067* | 0.058* |
| pos 1 | 1.911 | 2.608 |
| pos 2 | 0.299 | 0.375 |

| | | |
|---|---|---|
| * = scanning signal (Vmm) | | |

Table 3 shows that the uptake is decreased to some extent for strips having discrete application zones compared with addition to a single zone. The decrease is, however, marginal and is probably due to the fact that the flow rate in simultaneous application was somewhat delayed. The experiment therefore demonstrates that basically the same results may be achieved for simultaneous application to the zone sequence LZ₅B, LZ₄R*, LZ₃B, LZ₂S, LZ₁B as if sample, Reactant* and buffer are applied sequentially to a common application zone.

## Claims

1. A method for determination of an analyte in a sample in a flow matrix by use of a transport flow of one or more biospecific affinity reactants, at least one of which is analytically detectable (Reactant*) and one is firmly anchored in the matrix (Reactant I), and the flow matrix comprises:
A) an application zone for liquid (LZ), containing buffer and sample and optionally one or more of the reactants, but not Reactant I,
B) a detection zone (DZ) located downstream of LZ with the firmly anchored reactant (Reactant I), and
C) optionally one or more zones in which any of the reactants has been pre-deposited,
wherein (i) the flow towards the detection zone is initiated by addition of the liquid with sample in the application zone LZS for transport of analyte and reactants towards the detection zone (DZ), and (ii) the amount of Reactant* bound to DZ is detected, the detected amount being related to the amount of analyte in the sample, **characterized in that**
I. the flow matrix comprises at least two application zones for liquid arranged substantially adjacent to each other 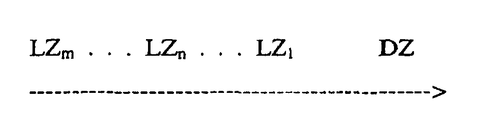 wherein
a) LZₙ is an application zone for liquid, and n is the position of the application zone LZₙ,
b) m is the total number of application zones in which flow is initiated (m ≥ 2),
c) one LZₙ is an application zone for sample (LZₙ,S) and one LZₙ is for Reactant* (LZ_{n"}R*) with n" ≥ n',
d) -----> is the direction of the flow, and
e) DZ is the detection zone, and
II. flow is initiated by adding liquid to each zone
LZₘ .. LZₙ .. LZ₁ (m≠n) in such a way that liquidₙ₊₁, added to the application zone LZₙ₊₁, contacts the flow matrix substantially simultaneously and is transported through the matrix immediately after liquidₙ, added to the nearest downstream application zone LZₙ.

2. The method according to claim 1, **characterized in that** n" > n' (sequential variants regarding analyte and Reactant*).

3. The method according to claim 1, **characterized in that** n" = n' (simultaneous variants regarding analyte and Reactant*).

4. The method according to any of the claims 1 - 3, **characterized in that** Reactant* is pre-deposited in its application zone (LZ_{n"}R*).

5. The method according to any of the claims 1 - 4, **characterized in that** LZₙ₊₁ finishes where LZₙ starts (m≠n).

6. The method according to any of the claims 1 - 5, **characterized in that** application of liquid is performed substantially simultaneously in all LZₘ .. LZₙ .. LZ₁.

7. The method according to any of the claims 1 - 6, **characterized in that** m ≤ 6; n' is 1, 2 or 3; n" > n'; LZ_{n'+1}, LZ_{n'+2}, LZ_{n'+3}, LZ_{n'-1}, and LZ_{n'-2} are application zones for liquids intended for transport of Reactant* or other reactant or buffer without reactant, as far as allowed by m, n" and n'.

8. The method according to any of the claims 1 - 7, **characterized in that** at least one of the zones
LZₘ . . LZₙ . . LZ₁ comprises a pad or material layer applied on the flow matrix.

9. The method according to any of the claims 1 - 7, **characterized in that** the zones LZₘ . LZₙ . LZ₁ have zone spacers between each other.

10. The method according to any of the claims 1 - 9, **characterized in that** the composition of transported components from an application zone LZₙ is not the same as from the nearest adjacent application zone LZ, in which flow is initiated, (LZₙ₊₁ and LZₙ₋₁, with the exception of n = m and n = 1, which zones lack LZₙ₊₁ and LZₙ₋₁, respectively).

11. The method according to any of the claims 1 - 10, **characterized in that** at least one reactant, other than Reactant*, is pre-deposited in an application zone LZ_{n'''}R for liquid intended for transport of the reactant.

12. The method according to any of the claims 1 - 11, **characterized in that** m ≤ 6 and that n' for the application zone for sample (LZ_{n'}S) is 1, 2 or 3.

13. The method according to any of the claims 1 - 12, **characterized in that** Reactant* has biospecific affinity for the analyte so that Reactant* is incorporated into a complex Reactant'---Analyte---Reactant* in the detection zone in an amount related to the amount of analyte in the sample, in which complex Reactant' has biospecific affinity to the analyte and is
(a) Reactant I, or
(b) a reactant to which Reactant I exhibits biospecific affinity and which is transported from LZ_{n'}S or from an application zone downstream of LZ_{n'}S.

14. The method according to any of the claims 1 - 13, **characterized in that** the matrix comprises at least one calibrator zone (CZ), in which calibrator is bound to, or in advance has been bound to the matrix.

15. The method according to claim 14, **characterized in that** the calibrator zone or zones (CZ) have a binder for the calibrator firmly anchored in the matrix, the calibrator optionally being pre-deposited in the matrix upstream of the calibrator zone or zones.

16. The method according to any of the claims 1 - 15, **characterized in that**
a. the analyte is chosen among antigens generally, and
b. the method is performed as part of diagnosing allergy or autoimmune disease.

## Patentansprüche

1. Verfahren zur Bestimmung eines Analyten in einer Probe in einer Durchflussmatrix unter Anwendung einer Transportströmung von einem oder mehreren biospezifischen Affinitätsreaktanten, von denen mindestens einer analytisch nachweisbar ist (Reaktant*) und einer fest in der Matrix verankert ist (Reaktant I), und wobei die Durchflussmatrix Folgendes umfasst:
A) eine Anwendungszone für Flüssigkeit (LZ), enthaltend Puffer und Probe und wahlweise einen oder mehrere der Reaktanten, aber nicht den Reaktanten I,
B) eine Nachweiszone (DZ), angeordnet stromabwärts der LZ mit dem fest verankerten Reaktanten (Reaktant I) und
C) wahlweise eine oder mehrere Zonen, in welchen einer der Reaktanten zuvor abgeschieden wurde,
wobei (i) die Strömung in Richtung der Nachweiszone durch die Zugabe der Flüssigkeit mit Probe zu der Anwendungszone LZS für den Transport von Analyt und Reaktanten in Richtung der Nachweiszone (DZ) initiiert wird und (ii) die Menge des an die DZ gebundenen Reaktanten* nachgewiesen wird, wobei die nachgewiesene Menge mit der Menge an Analyt in der Probe in Zusammenhang steht, **dadurch gekennzeichnet, dass**
I. die Strömungsmatrix mindestens zwei Anwendungszonen für Flüssigkeit umfasst, die im Wesentlichen benachbart zueinander angeordnet sind: 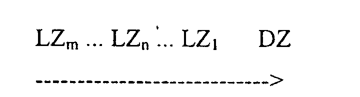 worin
a) LZₙ eine Anwendungszone für Flüssigkeit ist und n die Position der Anwendungszone LZₙ ist,
b) m die Gesamtzahl an Anwendungszonen ist, in welchen die Strömung initiiert wird (m ≥ 2),
c) eine LZₙ eine Anwendungszone für die Probe (LZ_{n'}S) ist und eine LZₙ für den Reaktanten* ist (LZ_{n"}R*), mit n" ≥ n';
d) -----> die Strömungsrichtung ist, und
e) DZ die Nachweiszone ist; und
II. die Strömung durch Zusetzen von Flüssigkeit zu jeder Zone
LZₘ .. LZₙ .. LZ₁ (m ≠ n) initiiert wird, in einer Weise, dass Flüssigkeitₙ₊₁, welche der Anwendungszone LZₙ₊₁ hinzugefügt wird, mit der Strömungsmatrix praktisch gleichzeitig in Kontakt kommt und durch die Matrix hindurch befördert wird unmittelbar nach der Flüssigkeitₙ, welche der nächsten stromabwärts gelegenen Anwendungszone LZₙ hinzugefügt wird.

2. Verfahren nach Anspruch 1, **dadurch gekennzeichnet, dass** n" > n' ist (aufeinanderfolgende Varianten bezüglich Analyt und Reaktant*).

3. Verfahren nach Anspruch 1, **dadurch gekennzeichnet, dass** n" = n' (gleichzeitige Varianten bezüglich Analyt und Reaktant*).

4. Verfahren nach mindestens einem der Ansprüche 1 - 3, **dadurch gekennzeichnet, dass** der Reaktant* in seiner Anwendungszone (LZ_{n"}R*) vorab abgeschieden wird.

5. Verfahren nach mindestens einem der Ansprüche 1 - 4, **dadurch gekennzeichnet,** das LZₙ₊₁ endet, wo LZₙ beginnt (m ≠ n).

6. Verfahren nach mindestens einem der Ansprüche 1 - 5, **dadurch gekennzeichnet, dass** die Anwendung von Flüssigkeit praktisch gleichzeitig in allen LZₘ .. LZₙ .. LZ₁ erfolgt.

7. Verfahren nach mindestens einem der Ansprüche 1 - 6, **dadurch gekennzeichnet, dass** m ≤ 6 ist; n' 1, 2 oder 3 ist; n" > n'; LZ_{n'+1}, LZ_{n'+2}, ; LZ_{n'+3}, LZ_{n'-1} und LZ_{n'-2} Anwendungszonen für Flüssigkeiten sind, die für den Transport des Reaktanten* oder eines anderen Reaktanten oder Puffers ohne Reaktant bestimmt sind, solange dies m, n" und n' zulassen.

8. Verfahren nach mindestens einem der Ansprüche 1 - 7, **dadurch gekennzeichnet, dass** mindestens eine der Zonen
LZₘ .. LZₙ .. LZ₁ ein Kissen oder eine Materialschicht, aufgebracht auf die Strömungsmatrix, umfasst.

9. Verfahren nach mindestens einem der Ansprüche 1 - 7, **dadurch gekennzeichnet, dass** die Zonen LZₘ .. LZₙ .. LZ₁ Zonen-Spacer zwischen diesen aufweisen.

10. Verfahren nach mindestens einem der Ansprüche 1 - 9, **dadurch gekennzeichnet, dass** die Zusammensetzung von transportierten Komponenten von einer Anwendungszone LZₙ nicht dieselbe ist wie von der nächstgelegenen Anwendungszone LZ, in welcher die Strömung initiiert wird (LZₙ₊₁ und LZₙ₋₁, mit der Ausnahme von n = m und n = 1, wobei den Zonen LZₙ₊₁ bzw. LZₙ₋₁ fehlen).

11. Verfahren nach mindestens einem der Ansprüche 1 - 10, **dadurch gekennzeichnet, dass** mindestens ein von dem Reaktanten* verschiedener Reaktant in einer Anwendungszone LZ_{n"'}R für Flüssigkeit, die für den Transport des Reaktanten bestimmt ist, vorab abgeschieden wird.

12. Verfahren nach mindestens einem der Ansprüche 1 - 11, **dadurch gekennzeichnet, dass** m ≤ 6 ist und dass n' für die Anwendungszone für die Probe (LZ_{n'}S) 1, 2 oder 3 ist.

13. Verfahren nach mindestens einem der Ansprüche 1 - 12, **dadurch gekennzeichnet, dass** der Reaktant* eine biospezifische Affinität zu dem Analyt besitzt, so dass der Reaktant* in einen Komplex Reaktant' ---Analyt---Reaktant* in der Nachweiszone in einer Menge in Verbindung mit der Menge an Analyt in der Probe eingebracht wird, in welcher der komplexe Reaktant' eine biospezifische Affinität zu dem Analyt besitzt und
(a) der Reaktant I ist, oder
(b) ein Reaktant, zu welchem der Reaktant I eine biospezifische Affinität besitzt und welcher von LZ_{n'}S oder von einer Anwendungszone stromabwärts von LZ_{n'}S transportiert wird.

14. Verfahren nach mindestens einem der Ansprüche 1 - 13, **dadurch gekennzeichnet, dass** die Matrix mindestens eine Kalibratorzone (CZ) umfasst, in welcher Kalibrator an die Matrix gebunden ist oder zuvor an diese gebunden wurde.

15. Verfahren nach Anspruch 14, **dadurch gekennzeichnet, dass** die Kalibratorzone oder -zonen (CZ) ein Bindemittel für den fest in der Matrix verankerten Kalibrator aufweisen, wobei der Kalibrator wahlweise in der Matrix vorab stromaufwärts der Kalibratorzone oder -zonen abgeschieden wird.

16. Verfahren nach mindestens einem der Ansprüche 1 - 15, **dadurch gekennzeichnet, dass**
a. der Analyt allgemein unter Antigenen gewählt ist, und
b. das Verfahren als Teil der Diagnose einer Allergie oder einer Autoimmunerkrankung durchgeführt wird.

## Revendications

1. Procédé de détermination d'un analyte dans un échantillon dans une matrice d'écoulement au moyen d'un écoulement de transport d'un ou plusieurs réactifs à affinité biospécifique, dont l'un au moins est détectable analytiquement (réactif*) et l'un est ancré solidement dans la matrice (réactif I), et la matrice d'écoulement comprend :
A) une zone d'application pour du liquide (LZ), contenant un tampon et un échantillon et éventuellement un ou plusieurs des réactifs, mais pas le réactif I,
B) une zone de détection (DZ) située en aval de LZ avec le réactif ancré solidement (réactif I), et
C) éventuellement une ou plusieurs zones dans lesquelles l'un quelconque des réactifs a été prédéposé,
où (i) l'écoulement en direction de la zone de détection est amorcé par addition du liquide avec l'échantillon dans la zone d'application LZS pour le transport de l'analyte et des réactifs en direction de la zone de détection (DZ), et (ii) la quantité de réactif* lié à DZ est détectée, la quantité détectée étant liée à la quantité d'analyte dans l'échantillon, **caractérisé en ce que**
I. la matrice d'écoulement comprend au moins deux zones d'application pour du liquide disposées sensiblement adjacentes l'une à l'autre : 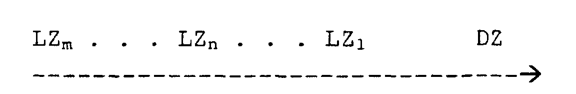 où
a) LZₙ est une zone d'application pour du liquide, et n est la position de la zone d'application LZₙ,
b) m est le nombre total de zones d'application dans lesquelles l'écoulement est amorcé (m ≥ 2),
c) une LZₙ est une zone d'application pour l'échantillon (LZ_{n'}S) et une LZₙ est pour le réactif* (LZ_{n''}R*) avec n" ≥ n',
d) -----> est la direction de l'écoulement et
e) DZ est la zone de détection, et
II. l'écoulement est amorcé par addition de liquide à chaque zone LZₘ . . L2ₙ . . LZ₁ (m≠n) de telle manière que le liquideₙ₊₁, ajouté à la zone d'application LZₙ₊₁, entre en contact avec la matrice d'écoulement sensiblement simultanément et est transporté dans la matrice immédiatement après le liquideₙ ajouté à la zone d'application en aval la plus proche LZₙ.

2. Procédé selon la revendication 1 **caractérisé en ce que** n'' > n' (variantes séquentielles concernant l'analyte et le réactif*).

3. Procédé selon la revendication 1 **caractérisé en ce que** n'' = n' (variantes simultanées concernant l'analyte et le réactif*).

4. Procédé selon l'une quelconque des revendications 1-3 **caractérisé en ce que** le réactif* est prédéposé dans sa zone d'application (LZ_{n''}R*).

5. Procédé selon l'une quelconque des revendications 1-4 **caractérisé en ce que** LZₙ₊₁ finit là où LZₙ commence (m ≠ n) .

6. Procédé selon l'une quelconque des revendications 1-5 **caractérisé en ce que** l'application de liquide est réalisée sensiblement simultanément dans toutes les LZₘ . . LZₙ . . LZ₁.

7. Procédé selon l'une quelconque des revendications 1-6 **caractérisé en ce que** m ≤ 6 ; n' est 1, 2 ou 3 ; n'' > n' ; LZ_{n'+1}, LZ_{n'+2}, LZ_{n'+3}, LZ_{n'-1} et LZ_{n'-2} sont des zones d'application pour des liquides destinés au transport du réactif* ou d'un autre réactif ou de tampon sans réactif, dans la mesure où m, n'' et n' le permettent.

8. Procédé selon l'une quelconque des revendications 1-7 **caractérisé en ce qu'**au moins l'une des zones LZₘ . . LZₙ . . LZ₁ comprend un tampon, ou une couche de matériau, appliqué sur la matrice d'écoulement.

9. Procédé selon l'une quelconque des revendications 1-7 **caractérisé en ce que** les zones LZₘ . LZₙ . LZ₁ ont des espaceurs de zones entre elles.

10. Procédé selon l'une quelconque des revendications 1-9 **caractérisé en ce que** la composition des composants transportés depuis une zone d'application LZₙ n'est pas la même que celle des composants transportés depuis la zone d'application adjacente la plus proche LZ, où l'écoulement est amorcé, (LZₙ₊₁ et LZₙ₋₁, à l'exception de n = m et n = 1, lesquelles zones sont dépourvues de LZₙ₊₁ et LZₙ₋₁, respectivement).

11. Procédé selon l'une quelconque des revendications 1-10 **caractérisé en ce qu'**au moins un réactif, différent du réactif*, est prédéposé dans une zone d'application LZ_{n'''}R pour du liquide destiné au transport du réactif.

12. Procédé selon l'une quelconque des revendications 1-11 **caractérisé en ce que** m ≤ 6 et **en ce que** n' pour la zone d'application pour l'échantillon (LZ_{n'}S) est 1, 2 ou 3.

13. Procédé selon l'une quelconque des revendications 1-12 **caractérisé en ce que** le réactif* a une affinité biospécifique pour l'analyte de sorte que le réactif* est incorporé dans un complexe réactif' --- analyte --- réactif* dans la zone de détection en une quantité liée à la quantité d'analyte dans l'échantillon, complexe dans lequel le réactif' a une affinité biospécifique pour l'analyte et est
(a) le réactif I ou
(b) un réactif pour lequel le réactif I présente une affinité biospécifique et qui est transporté depuis LZ_{n'}S ou depuis une zone d'application en aval de LZ_{n'}S.

14. Procédé selon l'une quelconque des revendications 1-13 **caractérisé en ce que** la matrice comprend au moins une zone d'étalon (CZ) où un étalon est lié à la matrice, ou a été lié à la matrice au préalable.

15. Procédé selon la revendication 14 **caractérisé en ce que** la zone ou les zones d'étalon (CZ) ont un liant pour l'étalon solidement ancré dans la matrice, l'étalon étant éventuellement prédéposé dans la matrice en amont de la zone ou des zones d'étalon.

16. Procédé selon l'une quelconque des revendications 1-15 **caractérisé en ce que**
a. l'analyte est choisi parmi les antigènes généralement, et
b. le procédé est mis en oeuvre dans le cadre du diagnostic d'une allergie ou d'une maladie auto-immune.
